Europäisches Patentamt

European Patent Office

Office européen des brevets

(19)

(11) Publication number: **0 044 611**

**A1**

(12)

# EUROPEAN PATENT APPLICATION

(21) Application number: **81302526.9**

(22) Date of filing: **08.06.81**

(51) Int. Cl.³: **A 43 D 1/02**

(30) Priority: **05.07.80 GB 8022108**

(43) Date of publication of application:
**27.01.82 Bulletin 82/4**

(84) Designated Contracting States:
**AT BE CH DE FR IT LI LU NL SE**

(71) Applicant: **P.R. Cooper (Footline) Limited**
**"Seaford House" 27 Stoughton Street**
**Leicester, LE2 0SH(GB)**

(72) Inventor: **Œ Cooper, Philip Roy**
**"Seaford House" 27 Stoughton Street**
**Leicester LE2 0SH(GB)**

(74) Representative: **Jones, Andree Zena et al,**
**E. N. Lewis & Taylor 144 New Walk**
**Leicester LE1 7JA(GB)**

(54) Gauge means for measurement of foot size, and methods of manufacture and use of the same.

(57) The invention relates to the measurement of foot size and shape for the accurate fitting of footwear, and is especially but not exclusively useful for measuring feet having abnormalities. A plastics gauge member (2), preferably of transparent sheet material, is vacuum formed to conform with a last bottom, and the fit of a shoe made on that last is confirmed by insertion of the patient's foot (16) into a recess (6, 10) of the gauge member. A forepart gauge member (12) may also be used. The fitter may observe pressure points through the transparent sheet material where these are caused by foot deformities (18), and mark the locations of these on the gauge to assist in an accurate and comfortable fitting.

FIG.1

Croydon Printing Company Ltd.

Title:   Gauge means for measurement of foot size, and methods of manufacture and use of the same

The invention relates to the measurement of foot size to facilitate the fitting of footwear of the correct internal dimensions.

The accurate fitting of footwear is a skilled operation which presents particular difficulties when the intending wearer is either a child or an elderly person. A young child is incapable of determining for itself whether a shoe fits correctly or not, and it is frequently difficult to determine the exact position of the child's foot in the shoe, despite initial use of a conventional foot gauge. Visual checks using X-rays are not generally accceptable because of radiation risks.

Difficulties also arise with accurate fitting of footwear for elderly people who have foot defects associated with old age, arthritis, the effects of ill-fitting shoes or a variety of other reasons. Many are unable readily to visit shoe shops, for example, because of their foot defects, and it is often necessary, especially in the case of those with severely deformed feet, for the footwear to be made individually and for a skilled fitter to make several journeys to a person's residence before the footwear is satisfactory. This makes the footwear extremely expensive to produce, and inevitably some errors in fitting are irremediable, and the shoes are useless. It will be understood that although the fitter could in theory carry a range of footwear which may be tried on by the intending wearer, each size would have to be provided in at least three widths and passibly three girths in order to provide any chance of one being found an accurate fit. Fitting problems are further complicated where the wearer is unable to communicate because of mental or other difficulties.

The object of the invention is therefore to provide a means whereby a fitter may readily ascertain the exact size and shape of a person's foot in order to provide accurately-fitting footwear, without having recourse to an empirical method involving unmanageably large numbers of sample items of footwear.

A further object of the invention is to provide a means whereby the exact position of abnormalities in foot structure may be ascertained so that such abnormalities may be compensated for or other steps taken to relieve the adverse effects thereof, when accurately-fitting footwear is being provided.

The invention therefore provides a gauge means for use in comparing the size and shape of a person's foot with respect to a footwear last, comprising a first gauge member having a recess formed therein having a base portion corresponding in plan to at least a portion of a sole surface of the last, wall portions of said recess being upstanding from said base portion in order to confront at least side surfaces of a foot placed in said recess for comparison purposes.

Advantageously the recess walls are provided with out-turned lip portions forming a peripheral flange extending around at least a portion of the length of the walls. In an example to be described below, a gauge member is formed to correspond to the entire sole surface of the last, with continuous recess walls around the length of the featherline and a continuous out-turned peripheral flange which serves to facilitate handling and accurate location during use.

Conveniently, the gauge means further comprises a second or subsequent gauge member adapted to co-operate with said first gauge member and having a foot-confronting surface shaped to follow the contour of an upper surface of the last said surface being bounded at least in part by a peripheral flange arranged in use to contact appropriate portions of the peripheral flange of the first gauge member. This may, by way of example, be a forepart gauge member.

The first and second gauge members may be formed from deformable plastics sheet material, which may advantageously be non-opaque. A batch of various sizes of gauge members may be adjustably connected together to form a graded set.

In use, a fitter will select a first gauge member approximating in size and width to the size required by the customer or patient. He will then place the patient's foot in the recess of the gauge member and will be then able to judge the accuracy of the size of a shoe made upon a last corresponding to that gauge member.

While this may be sufficient to ensure an accurate fit in many instances, for example with a child or a person having no foot abnormalities, it will often be necessary to check the foot size still further by employing a second gauge member, for example corresponding to the forepart of the last. This will be placed over the forepart of the patient's foot, so that the peripheral flange of the second gauge accurately confronts and contacts a peripheral flange of the first gauge member. It is likely, especially where toe deformities are  severe involving dislocations of the toe joints, that there will be insufficient height in a shoe made on a normal last. Thus the fitter will select a deeper forepart gauge member, ensuring that the finished shoe will not exert undue pressure on the toes.

Where the gauge members are of translucent or transparent material, the fitter is able to see whether there is undue pressure or not by viewing the appropriate region of the patient's foot through the gauge member(s).

If the deformity is such that, in the manufacture of the shoe, additional measures have to be taken to relieve possible pain caused through, say, pressure upon a dislocated joint, the fitter will observe the region of contact with the dislocated bone end through the gauge member and will mark the region upon the plastics surface. When the shoe is made, additional width or height may be given to the last at that region or, alternatively, padding material may be built into for example a resilient or padded insole so as to lie alongside the contact region in order to relieve the pressure upon the painful area.

The invention will now be described in detail with reference to the drawings. It will be understood that the description is given by way of example only and not by way of limitation.

In the drawings:-

Figure 1 is a perspective view of a main gauge according to the invention;

Figure 2 is a diagrammatic view in cross-section of a step in the formation of the gauge of Figure 1;

Figure 3 is a cross-sectional view of a last with a main gauge and a fore-part gauge;

Figure 4 is a fragmentary longitudinal section on line IV-IV of Figure 3; and

Figure 5 shows a step in the fitting operation using the gauges of Figure 3 and 4.

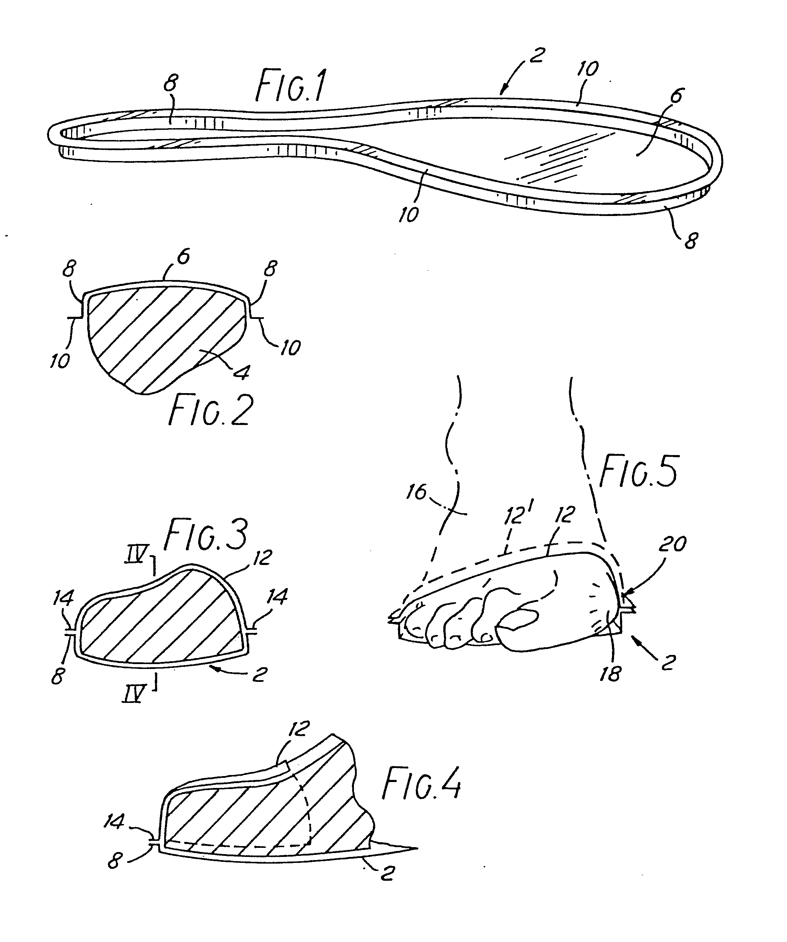

Figure 1 shows a first gauge member indicated by the reference numeral 2. The gauge member is formed from thin transparent plastics sheet material which has been shaped to form a recess which follows the appropriate contours of a last 4 (Figure 2), and comrises a base portion 6, upstanding wall portions 8 and an outwardly extending peripheral flange portion 10.

The gauge member 2 is shaped to the last contours by a vacuum-forming operation in which marginal portions of an over-size flat sheet of the plastics material are clamped in a machine (not shown) and by the application of heat and suction, the central portions of the sheet are drawn about the last in the region of the sole, the featherline and at least partly about the sides of the last as shown diagramatically in Figure 2.

A second, forepart, gauge member 12 having a flange 14 is formed in a similar manner by drawing a sheet of plastics material to conform with the vamp region of the last.

A set of the first gauge membrs may be assembled by securing a plurality thereof together with a flexible securing means. The gauge members are selected so as to be of graded dimensions and to fit one within the other for compactness. For example, gauge members 2 may be required for a size range of from 3 to 8 (adult female) or from 7 to 11 (adult male) in three widths C, D, E. A set of E fitting gauge members may be assembled with the size 7 gauge member resting within the contours of the size 8 gauge member and so on. The forepart gauge members may be similarly arranged in sets, if desired, to cover a range of variation of girth in each width.

In use, the intending wearer placed his foot 16 into the recess of the gauge member 2 selected by the fitter, the correct gauge member being the smallest one within the wall portions 8 of which the foot is comfortably received. Selected forepart gauge members 12 are then placed in turn over the foot until a reasonable fit is achieved when the flanges 8 and 14 are in a closely contactive position. When the foot has a particularly prominent feature, for example a bunion 18, the fitter marks the exact position of the feature on the transparent gauge member at 20 with, for example, a felt-tip pen or other marking means which permits eventual removal. Where the prominent feature is, for example, a hammer toe, it may be preferable to select a forepart gauge of greater height (as shown at $12^1$ in Figure 5). Thus, in the manufacture of the footwear from the fitting data, extra allowance may be made to give room for the prominent feature by adding suitable layers of, say, leather to the surface of last corresponding to the gauges. Thus when the shoe is made on that last, it will be an accurate fit with little or no further adjustment.

When the wearer is a child, it will usually be necessary only to use a first gauge member 2, unless the child's foot is deformed.

CLAIMS:

1. Gauge means for use in comparing the size and shape of a person's foot with respect to a footwear last, comprising a first gauge member having a recess formed therein having a base portion corresponding in plan to at least a portion of a sole surface of the last, wall portions of said recess being upstanding from said base portion in order to confront at least side surfaces of a foot placed in said recess for comparison purposes.

2. Gauge means as claimed in claim 1, wherein said recess walls are provided with out-turned lip portions forming a peripheral flange extending around at least a portion of the length of the walls.

3. Gauge means as claimed in claom 2, further comprising a second or subsequent gauge member adapted to co-operate with said first gauge member and having a foot-confronting surface shaped to follow the contour of an upper surface of the last, said surface being bounded at least in part by a peripheral flange arranged in use to contact appropriate portions of the peripheral flange of the first gauge member.

4. Gauge means as claimed in any one of claims 1 to 3, formed from deformable plastics sheet material.

0044611

5. Gauge means as claimed in claim 4, wherein a plurality of gauge members of graded sizes are adjustably connected together to form a graded set.

6. Gauge means as claimed in claim 4 wherein the plastics sheet material is non-opaque and has a surface capable of accepting ink or other similar markings.

7. A method of checking the size and shape of a person's foot with respect to a footwear last comprising the steps of taking gauge means as claimed in claim 6, placing the or each gauge member against the appropriate region of a person's foot and viewing the foot region through the material of the gauge portion to observe areas of above average pressure by the foot against the gauge member, and marking the material in those areas with ink or other similar marking.

8. A method of producing gauge means as claimed in claim 4 in so far as it is dependent on claim 2, involving the steps of placing a piece of heat-deformable plastics material against the sole of the last so as to cover at least a portion thereof, marginal portions of said sheet material extending outwardly beyond the featherline of said last, gripping peripheral portions of said marginal portions and causing the sheet to conform with the contours of the last in the region of the sole and featherline thereof, drawing the marginal portions over said featherline and against side walls of the last to form walls of the recess and rendering permanent the deformation of the plastics sheet material, the peripheral portions of the sheet material when released from being gripped forming the peripheral flange.

9. A method as claimed in claim 8, wherein said deformation is brought about by a vacuum forming step.

FIG.1

FIG.2

FIG.3

FIG.4

FIG.5

## European Patent Office

## EUROPEAN SEARCH REPORT

| DOCUMENTS CONSIDERED TO BE RELEVANT | | | CLASSIFICATION OF THE APPLICATION (Int Cl.3) |
|---|---|---|---|
| Category | Citation of document with indication, where appropriate, of relevant passages | Relevant to claim | |
| X | US - A - 3 022 577 (ROCKMORE)<br><br>* figure 1; column 2, lines 25-30 *<br><br>--- | 1,4,5,<br>8,9 | A 43 D 1/02 |
| X | US - A - 3 281 939 (McGINNITY)<br><br>* claims *<br><br>--- | 1,3-6 | |
| X | FR - A - 2 103 740 (I.G.I.S.P.A.)<br><br>* figure 1 *<br><br>--- | 1,2,4,<br>5 | **TECHNICAL FIELDS SEARCHED (Int. Cl.3)**<br><br>A 43 D 1/00<br>A 61 B 5/00 |
| X | GB - A - 1 077 879 (G.B. BRITTON)<br><br>* figures 1,2 *<br><br>--- | 1,2,4,<br>5 | |
| X | GB - A - 1 094 416 (G.B. BRITTON)<br><br>* figures 1-2 *<br><br>--- | 1,5 | |
| | FR - A - 605 599 (PFERSDORF)<br><br>* abstract *<br><br>------- | 1,6 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&. member of the same patent family, corresponding document

The present search report has been drawn up for all claims

| Place of search<br>The Hague | Date of completion of the search<br>06-10-1981 | Examiner<br>LOKERE |
|---|---|---|

EPO Form 1503.1  06.78